# EUROPEAN PATENT APPLICATION

(11) **EP 1 449 541 A1**
(43) Date of publication of application: **25.08.2004**
(21) Application number: 02783577.6
(22) Date of filing: 20.11.2002
(51) Int. Cl.: A61K 48/00, A61K 31/7088, A61K 45/00, A61K 41/00, A61K 47/12, A61K 47/26, A61K 47/34, A61K 47/42, A61K 47/44, A61P 37/06, A61P 43/00

(54) **COMPOSITIONS INHIBITING REJECTION IN ORGAN TRANSPLANTATION AND METHOD OF USING THE SAME**

(30) Priority: 22.11.2001 JP 2001358587
(71) Applicant: Anges MG, Inc., Toyonaka-shi, Osaka 560-0082 (JP)
(72) Inventor: MORISHITA, Ryuichi, c/o AnGes MG, Inc., Toyonaka-shi, Osaka 560-0082 (JP); TOMITA, Naruya, c/o AnGes MG, Inc., Toyonaka-shi, Osaka 560-0082 (JP); OGIHARA, Toshio, c/o AnGes MG, Inc., Toyonaka-shi, Osaka 560-0082 (JP); HIGASHI, Naoto, c/o AnGes MG, Inc., Toyonaka-shi, Osaka 560-0082 (JP)
(74) Representative: Williams, Aylsa
(86) International application number: PCT/JP2002/012142
(87) International publication number: WO 2003/043663

(57) **Abstract**

A novel and effective strategy for organ transplantation is provided. A therapeutic agent and method for preventing an acute rejection in a transplanted organ and improving prognosis are provided. A therapeutic agent for suppressing a rejection in organ transplantation, which comprises an NF-κB decoy compound, is provided. Representatively, the organ transplantation is kidney transplantation. The therapeutic agent further comprises an ultrasonic inspection contrast agent. A therapeutic agent for improving prognosis in organ transplantation is also provided. The therapeutic agent is administered into a donor organ. The transfection efficiency of the NF-κB decoy compound into the organ may be enhanced by ultrasound treatment.

## Description

### TECHNICAL FIELD

The present invention relates to a composition comprising a compound (e.g., a nucleic acid and a homolog thereof) which specifically binds to a site on a chromosome to which a transcriptional regulatory factor binds, and a method of using the same. More particularly, the present invention relates to a composition comprising a decoy compound, and a method of using the same.

### BACKGROUND ART

Acute allogenic rejection is an immunological phenomenon mediated by T-cells, in which a number of inflammatory mediators and adhesion molecules are involved [Hancock, 1983]. The onset of the complicated event requires the synergistic activation of multiple transcription factors [Shannon, 1995].

NF-κB is one of such transcriptional regulatory factors for genes encoding gene products important for inflammation and immune [Baeuerle, 1994]. NF-κB responds to various extracellular signals and rapidly migrates from the cytoplasm to the nucleus, and plays a pivotal role in the coordinated transactivation of several cytokines and adhesion molecule genes. Cooper et al. demonstrated a time-dependent increase in the DNA binding activity of NF-κB, which had a peak three days before rejection in an allogenic heart transplantation model [Cooper, 1998]. However, administration of PDTC which is a potent inhibitor for NF-κB reduced the NF-κB activity peak in the model, significantly elongating the survival of the recipient.

Novel immonosuppresants, FK506 and CsA, upregulate gene transcription by inhibiting calcineurin (a signal transducing phosphatase which is a key factor involved in the activation of NF-κB) [Mattila, 1990; McCaffrey, 1994; Kanno, 1995]. It has been explained that the effect of another major immunosuppresant, glucocorticoid, partly results from the inhibition of the activation of NF-κB [Scheinman, 1995; Auphan, 1995].

The normal active form of human NF-κB is a heterodimer of two DNA binding subunits, 50 kDa subunit (p50) and 65kDa subunit (relA or p65) [Lenardo, 1989; Libermann, 1990; Satriano J, 1994; Brennan, 1990; Neish, 1992]. In a cell which is not stimulated, NF-κB binds to an inhibition molecule known as IκB and hides within the cytoplasm. After a cell is stimulated, IκB is phosphorylated and then rapidly degraded. Thereafter, NF-κB is released from IκB, thereby making it possible to translocate the transcription factor to the nucleus, in which the transcription factor binds to various DNA recognition sites to regulate gene expression (Baeurerle, 1994, supra). It has been suggested that the dissociation of the transcription factor NF-κB from the complex induces regulated transactivation of genes including interleukins (ILs)-1, -6, and -8; intracellular adhesion factors; vascular cell adhesion factors; and endothelial cell adhesion factors, and plays a pivotal role in regulation of inflammatory changes [Lenardo, 1989; Libermann, 1990; Satriano J, 1994; Brennan, 1990; Neish, 1992; Yamazaki, 1993]. Therefore, blockage of NF-κB may attenuate gene-mediated cardiac ischemia-reperfusion.

A synthetic oligonucleotide acts as a cis-element "decoy compound" (hereinafter referred to as ODN), blocking a nuclear factor from binding to the promoter region of its intended gene, thereby inhibiting gene transactivation of *in vitro* and *in vivo* assay systems [Sullenger, 1990; Bienlinska, 1990; Yamada, 1995; Morishita, 1996]. Such a decoy strategy has been proposed for treatment of certain human diseases. The present inventors previously reported that transfection with E2F decoy ODN as a gene therapy model for restenosis inhibited neointimal proliferation after balloon-injury [Morishita, 1995]. Recently, the present inventors succeeded in *in vivo* protection of myocardiac muscle from ischemic injury using a decoy for NF-κB in rats.

### DISCLOSURE OF THE INVENTION

An object of the present invention is to prevent an acute rejection in organ transplantation, thereby improving prognosis.

The present inventors hypothesized that synthetic double-stranded DNA having high affinity for NF-κB, which is introduced *in vivo* as a cis-element decoy compound, binds to a transcription factor and blocks the activation of a gene mediating acute allogenic response, thereby providing an effective therapy for renal acute rejection.

The present inventors found that a sufficient amount of decoy ODN comprising an NF-κB cis-element, which was transfected into endothelial cells of a donor kidney, effectively bound to NF-KB, thereby preventing trans-activation of gene expression of essential cytokines and adhesion molecules, so that the establishment or progression of an acute rejection phenomenon was prevented. Thus, the present invention was completed.

Further, the present inventors found that by applying ultrasound exposure with the use of an echocardiographic contrast agent, Optison (trademark: Molecular Biosystems, Inc., USA) in perfusing solution, a sufficient amount of decoy ODN comprising NF-κB cis-element was successfully transfected into rat renal allografts. Thus, the present invention was completed.

The present invention relates to a therapeutic agent which suppresses a rejection in organ transplantation. The therapeutic agent comprises an NF-κB decoy compound. Examples of target organs include, but are not limited to, kidney, heart, lung, liver, spleen, pancreas, cholecystis, stomach, small intestine, large intestine, bladder, and the like.

Preferably, the above-described rejection is an allogenic response.

Preferably, the above-described allogenic response is acute.

Preferably, the above-described organ transplantation is kidney transplantation.

Preferably, the above-described therapeutic agent further comprises an ultrasonic inspection contrast agent.

Preferably, the above-described ultrasonic inspection contrast agent comprises a substrate selected from the group consisting of galactose, albumin, galactose and palmitic acid, liposome, polymer film, fatty acid, and lactic acid polymer. Examples of such an ultrasonic inspection contrast agent include Echovist (registered trademark) (Schering), Alubunex (registered trademark) (Molecular Biology, Inc.), Levovist (registered trademark) (Schering), DMP115 (Imagent) (Dupon Merk), EchoGen (registered trademark) (Sonus), Sonovist (registered trademark) (Schering), Sono Vue (registered trademark) (Bracco), BY963 (registered trademark) (Byk-Gulden), NC100100 (Nycomed), PESDA, Quantison (registered trademark) (Andaris), Quantison Depo (registered trademark) (Andaris), QUC82755, and the like.

Preferably, the above-described ultrasound inspection contrast agent is Optison (registered trademark). Optison is microbubble-containing small spheres derived from albumin, which comprise perfluorocarbon, which is an inert gas.

The present invention also relates to a therapeutic agent for improving prognosis in organ transplantation. The therapeutic agent comprises an NF-κB decoy compound.

The therapeutic agent is administered into a donor organ, and a transfection efficiency of the NF-κB decoy compound into the organ is enhanced by ultrasound treatment.

The present invention also relates to a method for suppressing a rejection in organ transplantation, comprising the steps of administering a therapeutic agent comprising a decoy compound into a donor organ, and subjecting the donor organ containing the decoy compound to ultrasound treatment.

The present invention also relates to a method for improving prognosis in organ transplantation, comprising the steps of administering a therapeutic agent comprising a decoy compound into a donor organ, and subjecting the donor organ containing the decoy compound to ultrasound treatment.

The present invention also relates to a method for enhancing transfection of an oligonucleotide into biological tissue, comprising the steps of introducing the oligonucleotide into the biological tissue, and subjecting the biological tissue containing the oligonucleotide to ultrasound treatment.

Preferably, the above-described ultrasound treatment is performed in the presence of an ultrasonic inspection contrast agent.

### BEST MODE FOR CARRYING OUT THE INVENTION

Hereinafter, embodiments of the present invention will be described. The embodiments described below are for illustrative purposes only and are not intended to limit the scope of the present invention.

The term "decoy" or "decoy compound" refers to a compound which binds to a site on a chromosome, which a transcriptional factor, such as NF-κB, binds to, or a site on a chromosome, which another transcription regulatory factor for a gene controlled by a transcriptional factor, such as NF-KB (hereinafter referred to as a target binding site) to antagonize the binding of NF-κB or other transcriptional factors to these target binding sites. Representatively, the decoy or the decoy compound is a nucleic acid or an analog thereof.

When a decoy is present within a nucleus, the decoy conflicts with a transcription regulatory factor competing for a target binding site for the transcription regulatory factor. As a result, a biological function which would be generated by binding of the transcription regulatory factor to the target binding site is inhibited. The decoy contains at least one nucleic acid sequence capable of binding to a target binding sequence. A decoy can be used for preparation of a pharmaceutical composition according to the present invention as long as the decoy has activity to bind to a target binding sequence.

Examples of preferable decoys include 5'-CCT TGAAGG GAT TTC CCT CC-3' (SEQ ID NO: 1) (NF-κB decoy), or oligonucleotide containing complementary sequences thereof, mutants thereof, or compounds containing these molecules therein. The oligonucleotides may be either DNA or RNA. The oligonucleotides may also include a modified nucleic acid and/or pseudonucleic acid therein. Further, these oligonucleotides may be mutants thereof, or compounds containing them therein. The oligonucleotides may have a single strand or double strands, or may be linear or circular. The mutants mean nucleic acids having the above-described sequences, a part of which has a mutation, a substitution, an insertion, or a deletion, and which specifically antagonize a transcriptional factor, such as NF-κB, or another transcription regulatory factor for a gene controlled by a transcriptional factor, such as NF-κB, with respect to the nucleic acid binding site to which the factor binds.

More preferable examples of the decoy include double-strand oligonucleotides containing one or a plurality of the above-described nucleic acid sequences, or mutants thereof. Nucleic acids containing one or a plurality of the above-described nucleic acid sequences are called chimeric (double) decoy when the number of nucleic acid sequences contained is two or triple decoy when the number of nucleic acid sequences contained is three, indicating the number of nucleic acid sequences.

The oligonucleotides for use in the present invention include oligonucleotides modified so as to resist *in vivo* degradation, and the like, such as oligonucleotides (S-oligo) having a thiophosphatediester bond which is a phosphatediester bond whose oxygen atom is replaced with a sulfur atom, oligonucleotides whose phosphatediester bond is substituted with a methylphosphate group having no electronic charge, and the like.

The decoy of the present invention can be produced with chemical or biochemical synthesis methods known in the art. For example, when a nucleic acid is used as a decoy compound, nucleic acid synthesis methods commonly used in genetic engineering can be employed. For example, a DNA synthesizer may be used to directly synthesize intended decoy nucleic acids. Further, these nucleic acids, nucleic acids containing the nucleic acids, or parts thereof may be synthesized, followed by amplification using a PCR method, a cloning vector, and the like. Furthermore, nucleic acids obtained by these methods are cleaved using a restriction enzyme, or the like, and linked or the like using DNA ligase, or the like to produce an intended nucleic acid. To obtain decoy nucleic acids which are more stable in cells, base, sugar and phosphate portions of the nucleic acids may be subjected to chemical modification, such as alkylation, acylation, or the like.

The therapeutic agent of the present invention may comprise a pharmaceutically acceptable carrier. Examples of such a pharmaceutically acceptable carrier include physiological saline, buffered physiological saline, dextrose, water, and the like. Generally, the pharmaceutically acceptable carrier is pharmaceutically inert.

The therapeutic agent of the present invention may comprise a decoy compound, an excipient, an adjuvant, and a pharmaceutically acceptable carrier. Further, the therapeutic agent may be administered into a patient in conjunction with other pharmaceutical agents in addition to the decoy compound.

The therapeutic agent of the present invention may be administered orally or parenterally. Parenteral administration includes local, topical, intra-arterial, intramuscular, subcutaneous, intramedullary, into subarachnoid space, intraventricular, intravenous, intraperitoneal, or intranasal administrations. Preferably, the composition of the present invention is administered by intravenous injection or intra-arterial injection. Techniques for prescription and administration are well known to those skilled in the art as described in "REMINGTON'S PHARMACEUTICAL SCIENCES" (Maack Publishing Co., Easton, PA).

The therapeutic agent of the present invention includes an agent containing an effective amount of decoy compound which can achieve the intended purpose of the decoy compound. Representatively, the therapeutic agent of the present invention comprises a decoy compound having a concentration of about 5 to 15 µM. "Therapeutically effective amount" or "pharmacologically effective amount" are terms which are well recognized by those skilled in the art and which refer to an amount of pharmaceutical agent effective for production of an intended pharmacological effect. Therefore, the therapeutically effective amount is an amount sufficient for reducing the manifestation of diseases to be treated. The therapeutically effective amount may be assayed by measuring the degree of recovery from a target disease. The therapeutically effective amount may depend on the condition of an individual to be treated. The amount may be optimized so as to achieve a desired effect without a significant side effect. The therapeutically effective amount may be determined using methods commonly used in the art. For example, a therapeutically effective amount may be estimated using a cell culture assay, an appropriate animal model, or the like. Thereafter, such information can be used to determine an amount and route effective for administration into humans.

The therapeutically effective amount of a decoy compound used in the present invention refers to an amount of the decoy compound which results in amelioration of symptoms or conditions of a disease. The therapeutic effect and toxicity of a decoy compound may be determined, for example, by standard pharmaceutical procedures in cell cultures or experimental animals (e.g., ED₅₀, a dose therapeutically effective for 50% of a population; and LD₅₀, a dose lethal to 50% of a population). Such a dose may vary depending upon the dosage form employed, the susceptibility of a patient, and the route of administration. Generally, a decoy compound is used at a concentration of 5 to 15 µM.

### BRIEF DESCRIPTION OF THE DRAWINGS

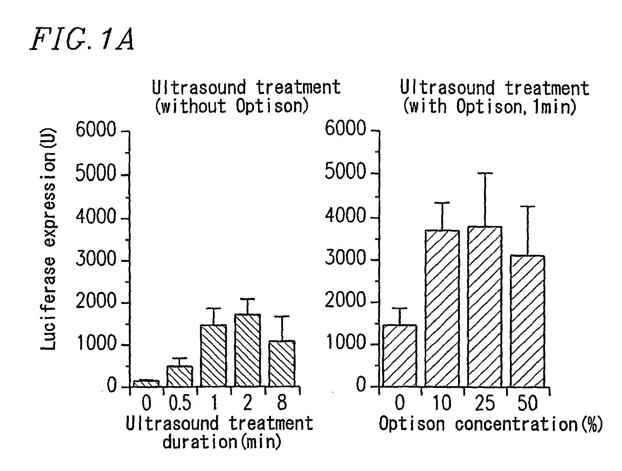

Figure **1A** shows a diagram indicating the efficacy of USE and Optison on gene transfection. The efficacy of USE on gene transfection was evaluated by a luciferase assay. The efficacy of ultrasound treatment (hereinafter referred to as USE) was proportional to its duration up to 2 min (a left-hand graph in Figure **1A**). Optison significantly enhanced gene transfection in a dose dependent manner (a right-hand graph in Figure **1A**).

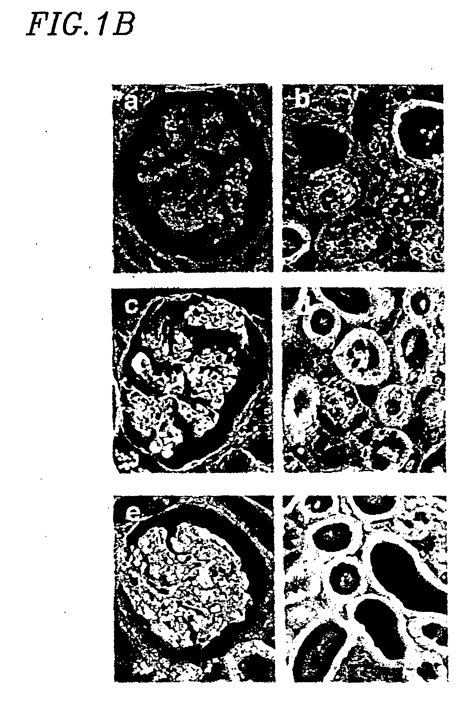

Figure **1B** shows photographs of sections of renal grafts indicating the results of evaluation of transfection of FITC-labeled NF-κB decoy ODN by fluorescent microscopy. The expression of FITC-staining was few in glomeruli (a), and slightly observed in tubules (b) with 30 sec of USE application without Optison. The expression increased both in glomeruli (c) and tubules (d) with 1 min of USE application and it was further enhanced by the use of Optison showing significant FITC-staining in glomeruli (e) and tubules (f) in the allografts.

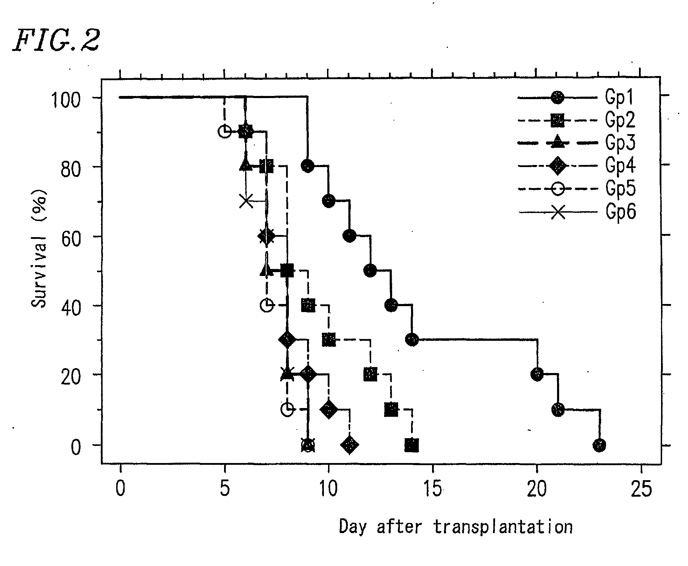

Figure 2 shows a graph indicating the survival of animals in a test. Animal survival was significantly prolonged with the treatment of donor kidneys through gene transfection of NF-κB decoy ODN with USE application (Group 1 and Group 2). In addition, the use of Optison enhances the efficacy of gene transfection, as evidenced by significantly prolonged animal survival in Group 1 as compared to all other groups; three out of ten animals survived for 20 days or more in this group. Recipients from Group 1 showed significantly prolonged survival as compared to all other groups; three out of ten animals survived for 20 days or more. These results indicate the efficacy of USE on gene transfection, and the use of Optison enhances the efficacy of USE on gene transfection. No significant difference between the survival time of recipients from Group 4 and Group 5, which indicates neither the addition of Optison in perfusing solution nor USE application influenced the animal survival.

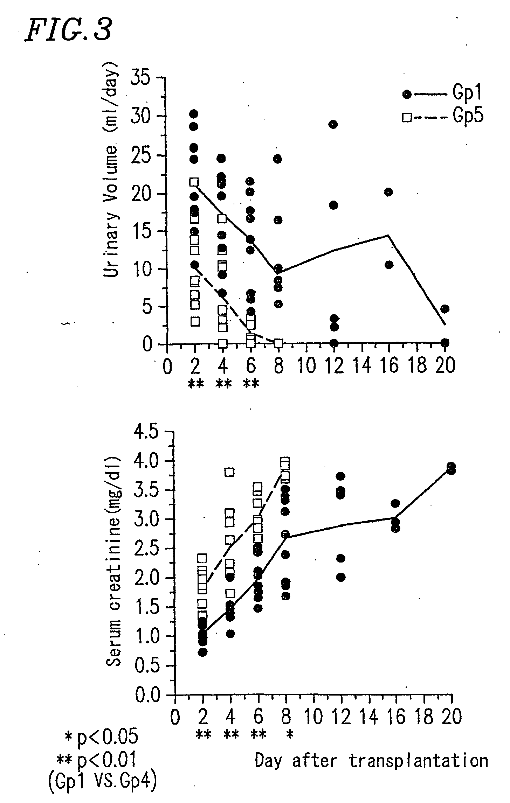

Figure 3 shows diagrams indicating the function of a renal graft in a test. The graft function was evaluated by the volume of urine (a) and the level of serum creatinine (S-Cr) (b). The urinary volume significantly decreased by day 2 after transplantation in recipients from Group 4 as compared to the recipients from Group 1. Most animals became anuria by day 6 after transplantation, while recipients from Group 1 kept a normal amount of urinary volume (a). The level of serum creatinine corroborated these results, showing a significantly elevated level of serum creatinine in the recipients from Group 4 as compared to that in the recipients from Group 1.

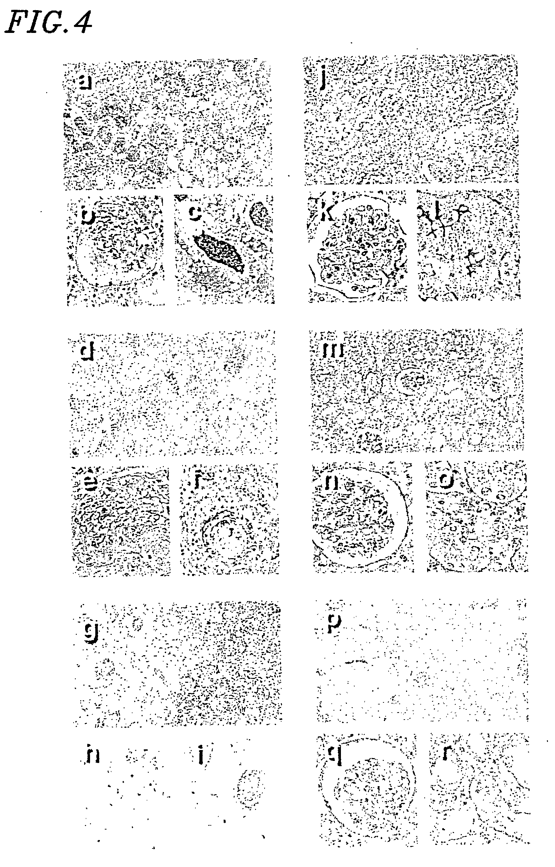

Figure **4** shows photographs of renal sections, indicating graft histology of a kidney in a test. Allograft organs from control animals harvested on day 2 after transplantation showed a large number of widespread mononuclear cell infiltrations (a). A number of glomeruli showed significant mononuclearcell infiltration with a large amount of mesangial matrix (b). Most tubules were structurally deformed with significant protein deposition and intratubular casts (c). Cellular infiltration had become florid in all areas with significant disruption of normal renal architecture and hemorrhage on day 4 (d). Some glomeruli (e) and tubules (f) showed severe structural deformity and necrosis with infiltration of a number of mononuclear cells. These changes underwent rapidly and aggressively by day 6, showing severe structural deformity with widespread necrotic tissue (g). Most glomeruli (h) and tubules (i) were replaced by severe necrosis. In contrast, allograft organs treated with NF-κB decoy were well preserved with few mononuclear cell infiltrations on day 2 (j). Most glomeruli showed no significant changes (k). Tubular structure remained mostly normal with no apparent protein deposition throughout the graft tissue (l). Only scant cellular infiltration was observed in interstitial areas by day 4 (m), and most glomeruli (n) and tubules (o) remained intact. On day 6, although a considerable number of cellular inflation appeared in the tissue (p), most glomeruli (q) and tubules (r) showed minor changes.

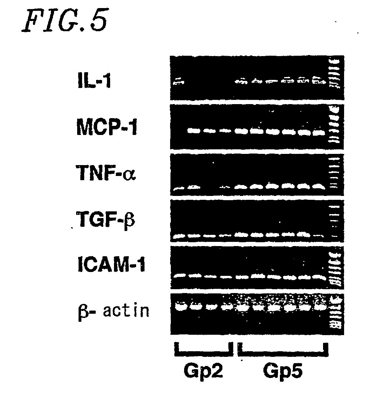

Figure 5 shows an electrophoresis photograph indicating the results of a reverse-transcription polymerase chain reaction (RT-PCR) assay.

Hereinafter, the present invention will be described by way of examples. The present invention is not limited to the examples. Materials, reagents, and the like used in the examples are available from commercial sources unless otherwise specified.

### EXAMPLES

The present invention will be described in greater detail by way of the following examples. Note that the following examples are provided for illustrative purposes and are not intended to limit the scope of the present invention.

### 1. Materials and Methods

### 1.1. Synthesis of ODN and Selection of Sequence Targets

Sequences of the phosphorothionate ODN used were as follows; NF-κB decoy ODN: 5'-CCT TGA AGG GAT TTC CCT CC-3' (SEQ ID NO.: 1), 3'-GGA ACT TCC CTA AAG GGA GG-5'; scrambled decoy ODN: 5'-TTG CCG TAC CTG ACT TAG CC-3' (SEQ ID NO.: 2), 3' -AAC GGC ATG GAC TGA ATC GG- 5' , andPRE (progesterone binding sequence): 5'-GAT CCT GTA CAG GAT GTT CTA GCT ACA-3' (SEQ ID NO.: 3), 3'-CTA GGA CAT GTC CTA CAA GAT CGA TGT-5'.

These ODNs were synthesized in accordance with commonly used methods. Synthetic ODNs were washed in 70% ethanol, dried, and dissolved in sterile Tris-EDTA buffer (10 mmol/L tris(hydroxymethyl)-aminomethane, 1 mmol/L ethylenediamine-tetraacetic acid). The supernatant was purified over NAP10 column (Parmacia, Sweden) and quantitatedbyspectrophotometry. NF-κB and scrambled decoy ODN were labeled with FITC at the 3' and 5' ends with an endo-labeling kit (Clonetech, Inc., Palo Alto Calif).

### 1.2. Acute rejection model in kidney transplantation

Inbred 200 to 250 g male Lewis rats (LEW, RTl^{l}) were used as graft recipients, and male WF (RTl^{u}) rats served as donors. The left kidney was removed from a WF rat after perfusingwithice-coldhepalinized saline (50 U/ml) through renal artery. The kidney was transplanted orthotopically to bilaterally nephrectomized Lewis recipientby end-to-end anastomosis of the left renal vessels and the left ureter using microsurgery technique. The technique is well established and the ischemic time is stable. During the procedure, the donor kidneys were subjected to 30 min of cold ischemic injury. All experimental protocols were conducted in accordance with the policies of the Animal Ethics Committee at the inventor's institution. In this model, animals typically suffer acute rejection, become anuric, and die from uremia within 10 days (CTLA-9). The survival time after the renal allograft is defined as the time from transplantation to the time of death. Animals dying from surgical technical failures within the first 24 h after transplantation were excluded from analysis.

### 1.3. Transfection of NF-κB decoy into donor kidney by applying ultrasound treatment with the use of an echocardiographic contrast agent (hereinafter referred to as USE), Optison, in perfusing solution

The present inventors performed the following study to determine the condition in which most transfection occurs in the donor kidney. The renal vein and ureter were clamped using a vascular clip after perfusion. Either 50 µg of a luciferase reporter gene or 100 µg of NF-κB decoy ODN labeled with FITC was dissolved in 0.5 ml of saline containing Optison at three different concentrations (0, 10, or 25%) and was infused into the kidney through the renal artery. The kidney was removed and exposed to ultrasound at a carrier frequency of 2 MHz in a water bath, where the signal intensity was in 4 bars and the exposure duration was varied from 30 sec to 8 min. The kidney was then transplanted into the Lewis recipient . The allograft organ was removed at day 1, 2 and 4, respectively, and the expression of a luciferase reporter gene and NF-κB decoy ODN was evaluated by a luciferase assay and fluorescence microscopy, respectively.

### (Experimental Design)

The present inventors made six experimental groups as listed in Table 1 to examine the efficacy of ultrasound treatment with the use of Optison and to establish the new procedure for the treatment of renal allograft organs using NF-κB decoy ODN.

**Table 1**

| | ODN (100 µg) | Opt (10%) | USE (1 min) |
|---|---|---|---|
| Group 1 (Gp1) | NF | ○ | ○ |
| Group 2 (Gp2) | NF | × | ○ |
| Group 3 (Gp3) | NF | ○ | × |
| Group 4 (Gp4) | NF | × | × |
| Group 5 (Gp5) | SD | ○ | ○ |
| Group 6 (Gp6) | × | × | × |

Group 1, 100 µg of NF-κB decoy ODN (NF) dissolved in 0.5 ml of saline containing 10% of Optison (Opt) was infused into each donor kidney. The gene transfection was performed by applying ultrasound treatment (USE) for 1 min; Group 2, the donor kidneys were treated with the same amount of NF-κB decoy with USE application, but without Optison; Group 3, the kidneys were treated with the same amounts of Optison and NF-κB decoy, but without USE application; Group 4, the kidneys were treated only with the same amount of NF-κB decoywithout Optison and USE application; Group 5, the kidneys were treated with the same amount of scrambled decoy containing Optison as that of NF-κB, with USE application; and Group 6, the donor kidneys were subjected to no treatment.

NF-κB decoy (100 µg) dissolved in 0.5 ml of saline with Optison at a concentration of 10% (Group 1: Gp1) or 100 µg of NF-κB decoy dissolved in 0.5 ml of saline without Optison (Group 2: Gp2) was infused into each donor kidney. The kidneys were subjected to USE at a carrier frequency of 2 MHz in a water bath for 1 min, and transplanted orthotopically into the Lewis recipients. The donor kidneys treated with the same amount of NF-κB decoy with Optison (10%) (Group 3: Gp3) or without Optison (Group 4: Gp4), were transplanted into the recipients without USE application. As a control group, donor kidneys treated with the same amount of scrambled decoy containing Optison with USE application were transplanted into recipients of group 5 (Gp5). Recipients of renal allografts without these treatments served as another control group (Group 6: Gp6). Animals were randomly selected from each group and evaluated for the graft organ survival. The survival time after the renal graft is defined as the time from transplantation to the time of death. Animals dying from surgical technical failures within the first24 h after transplantation were excluded from analysis. The donor kidneys of five animals randomly selected from each group were resected on day 4, and prepared for evaluation of ODN transfection, histological analysis, and immunohistology.

### (Graft organ function)

After transplantation, urine was collected at 24 hours on alternate days, the volume of the urine was measured serially, and the characteristics of the urine were tested by examining urinary sediment. The present inventors then assessed the function of the graft organ more quantitatively by measurement of serial serum creatinine levels in the recipients of from Group 1 to Group 5. Serum was obtained from the tail vein on alternate days, and serum creatinine (S-Cr) was measured by Jaffe reaction method.

### (Graft organ morphology)

Renal tissues were fixed in 4% paraformaldehyde in phosphate-buffered saline. Paraffin sections were stained with hematoxylin and eosin (HE) and periodic acid-Schiff (PAS), followed by assessment by light microscopy. Histological evaluation was performed on the basis of Banfu' s criteria.

Staining for ED1, CD4, or CD8 positive cells were performed by the alkaline phosphatase and anti-alkaline phosphates (APAAP) method using DAKO APAAP KIT (DAKO Japan, Kyoto, Japan) according to the manufacture's instructions. Monoclonal antibodies against ED1, CD4, or CD8 were purchased from Quantum Appligene (Parcd' innovation, illkirch, France). Staining for cytokines and adhesion molecules including IL1, IL2, IL6, monocyte chemoattractant protein-1 (MCP-1), TNF-α, intracellular adhesion molecule-1 (ICAM-1), and VCAM-1 was performed by the immunoperoxidase method. Anti-rat rabbit polyclonal antibodies against IL1, IL2, TNF-α, ICAM-1, VCAM-1 (Santa Cruz Biotechnology, Santa Cruz, CA), and MCP-1 (Pepro Tech, London, England) were used for primary antibodies. Biotinated anti-rabbit goat Ig (Santa Cruz Biotechnology, Santa Cruz, CA) was used as a secondary antibody. The reaction was visualized with 3,3'-diaminobenzidine.

The number of marker-positive cells was expressed as mean ± standard deviation (M ± SD) of cells per field of view (c/FV). Twenty or more fields of view were evaluated at a magnification of 400× for each section/specimen. The expression of adhesion molecules, cytokines and extracellular matrix was quantified on a 0 to 4+ scale (4+ = dense).

### (Reverse-Transcription Polymerase Chain Reaction (RT-PCR) Assay)

RNA was extracted from tissues using RNeasy Total RNA KitsR (QIAGEN, Hilden, Germany) according to the manufacturer's instructions. The quantity of RNA was confirmed on formaldehyde-agarose gel electrophoresis, and cDNA was prepared as described previously [Azuma, 2001]. PCR was performed by GeneAmp 9600 PCR system (Perkin-Elmer, Norwalk, CT), using primers for MCP-1, TNF-α, IL-6, inducible nitric oxide synthases (iNOS), and β-actin. Primer sequences, annealing temperature, and the number of cycles were as follows: MCP-1, 5' ATG CAG GTC TCT GTC ACG (SEQ ID NO.: 4 and 3' CTA GTT CTC TGT CAT ACT (55°C, 33 cycles); TGF-β1, 5' CTG CAG CTC CAC AGA GAA GAA CTG C and 3' CAC GAT CAT GTG GGA CAA CTG CTC C (SEQ ID NO.: 5) (64°C, 28 cycles); TNF-α, 5' TAC TGA ACT TCG GGG TGA TTG GTC C (SEQ ID NO.: 6) and 3' CAG CCT TGT CCC TTG AAG AGA ACC (60°C, 34 cycles); IL-1, 5' TGA TGT CCC ATT AGA CAG C (SEQ ID NO.: 7) and 3' GAG GTG CTG ATG TAC CAG TT (55°C, 35 cycles); IL-6, 5' CAA GAG ACT TCC AGC CAG TTG C (SEQ ID NO.: 8) and 3' TTG CCG AGT AGA CCT CAT AGT GAC C (30 cycles); iNOS, 5' TGC CAG GGT CAC AAC TTT ACA GG (SEQ ID NO.: 9) and 3' GGT CGA TGT CAC ATG CAG CTT GTC (35 cycles); ICAM-1, 5' AGA AGG ACT GCT GGG GAA (SEQ ID NO.: 10) and 3' CCT CTG GCG GTA ATA GGT G (60°C, 28 cycles); VCAM-1, 5' CTG ACC TGC TGC TCA AGT GAT GG (SEQ ID NO.: 11) and 3' GTG TCT CCC TCT TTG ACG CT (60°C, 26 cycles); β-actin, 5' TTG TAA CCA ACT GGG ACG ATA TGG (SEQ ID NO.: 12) and 3' GAT CTT GAT CTT CAT GGT GCT AGG (60°C, 23 cycles). DNA amplicons were electrophoresed on 1.5% agarose gels and visualized as bands under ultraviolet light with ethidium bromide staining (0.05 mg/ml for 10 min). The densities of competitive mimic and target cDNA were measured by scanning densitometry using SCANJET 4c (Hewlett Packerad, Corvallis, OR) with Adobe PHOTOSHOP software (Adobe Systems, Mountainview, CA). The ratios of densities of bands were plotted to establish a linear relationship over serial dilutions of template. Expression of mRNA was calculated based on the densities of sample and mimic amplicons: each result was expressed as a ratio of sample to β-actin. RNA was also subjected directly to amplification to exclude contamination by genomic DNA. The above-described manipulation was performed twice for each sample. The resultant values were expressed as mean ± standard deviation.

### (Statistical Analysis)

One-way analysis of variance (ANOVA) was performed on the values obtained for the urine volume and the serum creatinine. Graft organ survival was evaluated by the Kaplan-Meier test. The unpaired student's t-test was used for cellular infiltration in immunohistochemistry. Mann Whitney-U test was performed for histological analysis and data from expression of adhesion molecules, cytokines and extracellular matrix in immunohistochemistry. Results from RT-PCR were subjected to ANOVA without replication. If the ANOVA was significant, individual comparisons were made by the student's t-test. P value less than 0.05 was considered to be statistically significant.

### 2. Results

### 2.1. The condition in which the maximal gene transfection occurs without causing notable adverse reactions

The present inventors firstly examined the efficacy of USE on gene transfection into donor kidneys using a luciferase reporter gene and a FITC-labeled NF-κB decoy ODN. The results are shown in Figures **1A** and **1B**. USE significantly enhanced transfection of the luciferase reporter gene in proportion to the duration of USE up to 1 min as shown in a left-hand graph of Figure **1A**. The results from the expression of NF-κB decoy ODN demonstrated its efficacy (Figure **1B**).

Figure **1B** shows photographs of sections of a kidney transfected with FITC-labeled NF-κB decoy ODN, which evaluated by fluorescence microscopy. As shown in Figure **1B**, the results from the expression of NF-κB decoy ODN demonstrated its efficacy, showing significant FITC-staining in tubular cells in the allografts. Specifically, as shown in Figure **1B,** the expression of FITC-staining was few in glomeruli (a), and slightly observed in tubules (b) with 30 sec of USE application without Optison. The expression increased both in glomeruli (c) and tubules (d) with 1 min of USE application and it was further enhanced by the use of Optison showing significant FITC-staining in glomeruli (e) and tubules (f) in the allograft organs.

Referring again to Figure **1A**, significant tissue injury was noted when USE was performed for 2 min or more, while no significant injury was observed up to 1 min of USE application. Therefore, the present inventors set the duration of USE as 1 min. The present inventors then examined the efficacy of an echocardiographic contrast agent, Optison, with the use of USE. As shown in a right-hand graph of Figure **1A**, the use of Optison significantly enhanced gene transfection of the luciferase reporter gene in a dose dependent manner. However, significant tissue injury occurred when the concentration of Optison was 25%, while no significant injury was noted at 10%. Therefore, the present inventors decided the concentration of Optison in perfusing solution as 10% and the duration of USE application as 1 min.

### 2.2. Transfection of NF-κB decoy into donor kidneys prolonged the survival of animals

Figure 2 shows the results of the survival of animals in each group. The survival time in this model without any treatment was stable (mean ± SD = 7.5 ± 1.2, n=10); all animals died by day 9. In contrast, the recipients of donor kidneys treated with NF-κB decoy ODN using USE application (Group 1 and Group 2) showed prolonged animal survival as compared with all other groups. In addition, the use of the contrast agent, Optison, enhanced the efficacy of USE application on gene transfection, as evidenced by significantly prolonged animal survival in Group 1; three out of ten animals survived for 20 days or more in this group. These results indicate that the transfection of NF-κB decoy ODN using USE application into the donor kidney had a beneficial effect on the prolongation of graft survival and the use of Optison enhanced the efficacy of USE.

### 2.3. Graft function

Figure 3 shows the results of measurement of the function of graft organs in the test groups. The recipients of allografts treated with scrambled decoy (Group 5) showed significant hematuria by day 2 after transplantation. The volume of urine significantly decreased by this time as compared to that of recipients from Group 1 as shown in an upper graph of Figure 3 (mean ± SD = 9.77 ± 3.68 vs. 21.81 ± 6.85 ml/day, n=10). Most animals became anuria by day 6 after transplantation (1.29 ± 1.12 ml/day, n=9), while most recipients bearing allografts treated with NF-κB decoy kept a normal volume of urine without showing significant hematuria by day 6 (13.93 ± 6.42 ml/day, n=10).

As shown in a lower graph of Figure 3, the serum creatinine levels corroborated these results, showing significant elevated values in the recipients from Group 5 by day 2 after transplantation as compared to that of recipients from Group 1 (1.84 ± 0.23 mg/day vs. 0.97 ± 0.16 mg/day, n=10). Such data from graft function clearly confirmed that the animals died of renal failure and the prolongation of life was due to graft organ survival.

### 2.4. Significantly well preserved graft organ histology by NF-κB treatment

Figure **4** shows graft organ histology of each group. Allograft organs from control animals harvested on day 2 after transplantation showed a large number of widespread mononuclear cell infiltrations (a). A number of glomeruli showed significant mononuclear cell infiltration with a large amount of mesangial matrix (b). Most tubules were structurally deformed with significant protein deposition and intratubular casts (c). Cellular infiltration had become florid in all areas with significant disruption of normal renal architecture and hemorrhage on day 4 (d). Some glomeruli (e) and tubules (f) showed severe structural deformity and necrosis with infiltration of a number of mononuclear cells. Changes occurred rapidly and aggressively by day 6, showing severe structural deformity with widespread necrotic tissue (g). Most glomeruli (h) and tubules (i) were replaced by severe necrosis. In contrast, allograft organs treated with NF-κB decoy were significantly well preserved with few mononuclear cell infiltrations on day 2 (j). Most glomeruli showed no significant changes (k). Tubular structure remained mostly normal with no apparent protein deposition throughout the graft tissue (l). Only scant cellular infiltration was observed in interstitial areas by day 4 (m), and most glomeruli (n) and tubules (o) remained intact. On day 6, although a considerable number of cellular inflation appeared in the tissue (p), most glomeruli (q) and tubules (r) showed minor changes.

### 2.4. Reverse-Transcription Polymerase Chain Reaction (RT-PCR) Assay

Figure 5 shows the results of an RT-PCR assay. Figure 5 shows electrophoresis photographs indicating the results of the RT-PCR as say for Group 2 (NF-κB was administered, USE application) and Group 5 (scrambled decoy was administered, USE application). As shown in Figure **5**, the expression of IL-1, MCP-1, TNF-α, and TGF-β was observed in rats of Group 2, while the expression of TGF-β tended to be suppressed in rats of Group 5. Thus, it was demonstrated that the production of cytokines and the expression of adhesion molecules were suppressed in donor kidneys treated with NF-κB.

The above-described results will be summarized below.

The present inventors synthesized a fluorescence isothiocyanate (FITC)-labeled cis-element decoy compound against an NF-κB binding site (NF-κB decoy) and transfected Wister Firth (WF) kidneys with the decoy by applying ultrasound treatment (USE). In addition, an echographic contrast medium, Optison, further enhanced transfection with the use of USE, showing significant FITC-staining in tubular cells and glumeruli. Donor kidneys transfected with NF-κB decoy were orthotopically transplanted into bilaterally nephrectomized Lewis recipients. The recipients bearing donor kidneys treated with scrambled decoy (SD) instead of NF-κB decoy served as controls.

In the control group, significant destruction of renal tissue with a number of mononuclear cell infiltrations occurred on day 4. Immunohistology and RT-PCR demonstrated an increase in cytokine production and the expression of adhesion molecules in the grafted kidneys. All animals died of renal failure by day 10. In contrast, recipients having allografts transfected with NF-κB decoy showed prolonged survival, especially with USE application and the use of Optison (17.9 ± 8.3 (average number of days for survival) vs. 7.7 ± 1. 6 (average number of days for survival of control), n=10). Three out of ten animals survived for 20 days or more. Renal graft function and histology were well preserved (serum creatinine = 0.69 ± 0.12 vs. 2.45 ± 0.12 mg/dl (control) on day 4) with a significant decrease in cytokine production and the expression of adhesion molecules.

Thus, the new procedure, the application of USE with the use of Optison, enhanced the transfection of NF-κB decoy compound ODN into kidney grafts and improved animal survival associated with the inhibition of cytokine production and the expression of adhesion molecules. The new approach is a novel and effective strategy for renal allografts.

The above-described examples are provided for illustrating various aspects of the present invention and are not intended to limit the scope of the present invention.

### INDUSTRIAL APPLICABILITY

A novel and effective strategy for organ transplantation is provided. A therapeutic agent and method for preventing acute rejection to a graft organ and improving prognosis thereof is provided.

## Claims

1. A therapeutic agent for suppressing a rejection in organ transplantation, comprising an NF-κB decoy compound.

2. A therapeutic agent according to claim 1, wherein the rejection is an allogenic response.

3. A therapeutic agent according to claim 1, wherein the allogenic response is acute.

4. A therapeutic agent according to claim 1, wherein the organ transplantation is kidney transplantation.

5. A therapeutic agent according to claim 1, further comprising an ultrasonic inspection contrast agent.

6. A therapeutic agent according to claim 5, wherein the ultrasonic inspection contrast agent comprises a substrate selected from the group consisting of galactose, albumin, galactose and palmitic acid, liposome, polymer film, fatty acid, and lactic acid polymer.

7. A therapeutic agent according to claim 5, wherein the ultrasonic inspection contrast agent is Optison (registered trademark).

8. A therapeutic agent for improving prognosis in organ transplantation, comprising an NF-κB decoy compound.

9. A therapeutic agent according to claim 7, wherein the therapeutic agent is administered into a donor organ, and a transfection efficiency of the NF-KB decoy compound into the organ is enhanced by ultrasound treatment.

10. A method for suppressing a rejection in organ transplantation, comprising the steps of:
administering a therapeutic agent comprising a decoy compound into a donor organ; and
subjecting the donor organ containing the decoy compound to ultrasound treatment.

11. Amethodfor improving prognosis in organ transplantation, comprising the steps of:
administering a therapeutic agent comprising a decoy compound into a donor organ; and
subjecting the donor organ containing the decoy compound to ultrasound treatment.

12. A method for enhancing transfection of an oligonucleotide into biological tissue, comprising the steps of:
introducing the oligonucleotide into the biological tissue; and
subjecting the biological tissue containing the oligonucleotide to ultrasound treatment.

13. A method according to claims 10 to 12, wherein the ultrasound treatment is performed in the presence of an ultrasonic contrast agent.


SEQUENCE LISTING
<110> Anges M G, Inc.
<120> A composition for suppressing a graft rejection in an organ
transplantation and a method for using the same.
<130>
<160> 12
<170> PatentIn Ver. 2.1
<210> 1
<211> 20
<212> DNA
<213> Artificial Sequence
<220>
<223> Description of Artificial Sequence: NF-κB decoy
<300>
<400> 1
ccttgaaggg atttccctcc 20

<210> 2
<211> 20
<212> DNA
<213> Artificial Sequence
<220>
<223> Description of Artificial Sequence: Scramble decoy
<400> 2
ttgccgtacc tgacttagcc 20

<210> 3
<211> 27
<212> DNA
<213> Artificial Sequence
<220>
<223> Description of Artificial Sequence: PRE
<400> 3
gatcctgtac aggatgttct agctaca 27

<210> 4
<211> 18
<212> DNA
<213> Artificial Sequence
<220>
<223> Description of Artificial Sequence: MCP-1 Primer
<400> 4
atgcaggtct ctgtcacg 18

<210> 5
<211> 25
<212> DNA
<213> Artificial Sequence
<220>
<223> Description of Artificial Sequence: TGF-β1 Primer
<400> 5
ctgcagctcc acagagaaga actgc 25

<210> 6
<211> 25
<212> DNA
<213> Artificial Sequence
<220>
<223> Description of Artificial Sequence: TNF-α Primer
<400> 6
tactgaactt cggggtgatt ggtcc 25

<210> 7
<211> 19
<212> DNA
<213> Artificial Sequence
<220>
<223> Description of Artificial Sequence: IL-1 Primer
<400> 7
tgatgtccca ttagacagc 19

<210> 8
<211> 22
<212> DNA
<213> Artificial Sequence
<220>
<223> Description of Artificial Sequence: IL-6 Primer
<400> 8
caagagactt ccagccagtt gc 22

<210> 9
<211> 23
<212> DNA
<213> Artificial Sequence
<220>
<223> Description of Artificial Sequence: iNOS Primer
<400> 9
tgccagggtc acaactttac agg 23

<210> 10
<211> 18
<212> DNA
<213> Artificial Sequence
<220>
<223> Description of Artificial Sequence: ICAM-1 Primer
<400> 10
agaaggactg ctggggaa 18

<210> 11
<211> 23
<212> DNA
<213> Artificial Sequence
<220>
<223> Description of Artificial Sequence: VCAM-1 Primer
<400> 11
ctgacctgct gctcaagtga tcg 23

<210> 12
<211> 23
<212> DNA
<213> Artificial Sequence
<220>
<223> Description of Artificial Sequence: β-actin Primer
<400> 12
ttgtaaccaa ctgggacgat atgg 24